# EUROPÄISCHE PATENTANMELDUNG

(11) **EP 2 801 331 A1**
(43) Veröffentlichungstag der Anmeldung: **12.11.2014**
(21) Anmeldenummer: 13002480.5
(22) Anmeldetag: 10.05.2013
(51) Int. Cl.: A61B 17/86, A61B 17/88

(54) **Schraube, insbesondere Knochenschraube zur Verwendung in der Chirurgie**

(71) Anmelder: Bossard AG, 6301 Zug (CH)
(72) Erfinder: Grob, Beat, 6330 Cham (CH)
(74) Vertreter: Münch, Martin Walter

(57) **Zusammenfassung**

Die vorliegende Erfindung betrifft eine Schraube (1) umfassend einen Schraubenschaft (2), dessen Aussenfläche zumindest über einen Teilbereich seiner axialen Erstreckung von einem Gewinde (3) gebildet bzw. überzogen ist, und einen Schraubenkopf (4), welcher an einem Ende des Schraubenschafts (2) angeordnet ist und radial über den Schraubenschaft (2) übersteht. Die Schraube (1) weist eine Öffnung (5) auf, welche von der dem Schraubenschaft (2) abgewandten Seite des Schraubenkopfes (4) her in axialer Richtung in den Schraubenkopf (4) eindringt, diesen vollständig durchsetzt und in den Schraubenschaft (2) eindringt, welchen sie zumindest teilweise durchsetzt. Die Öffnung (5) weist in einem Bereich, welcher sich innerhalb des Schraubenschafts (2) erstreckt, einen nicht-kreisrunden Querschnitt auf, zur Ermöglichung eines rotatorischen Formschlusses mit einer entsprechenden Gegenkontur (8) eines Ausdrehwerkzeugs (7) zwecks Übertragung eines Drehmoments um die Schraubenlängsachse herum in Gewindeausdrehrichtung auf die Schraube (1).

Durch die erfindungsgemässe Ausgestaltung der Schraube (1) wird es möglich, auch beschädigte derartige Schrauben (1) in kürzester Zeit mit einem geeigneten Ausdrehwerkzeug (7) ohne Späne und/oder Abrieb erzeugende Eingriffe zu bergen. Insbesondere bei Arbeiten in Gefahrenzonen oder in der Chirurgie lässt sich hierdurch das Gefahren- bzw. Komplikationspotential deutlich reduzieren.

## Beschreibung

Die vorliegende Erfindung betrifft eine Schraube, insbesondere eine Knochenschraube zur Verwendung in der Chirurgie, ein Werkzeug zum Ein- bzw. Ausdrehen einer solchen Schraube, ein Set umfassend eine solche Schraube und ein solches Werkzeug sowie ein Verfahren zur spanlosen Entfernung einer insbesondere beschädigten solchen Schraube aus einem zumindest mit einem Teil des Schraubengewindes in Eingriff stehenden Material gemäss den Oberbegriffen der unabhängigen Patentansprüche.

Das Herausdrehen beschädigter Schrauben, z.B. mit rund gedrehtem oder abgerissenem Schraubenkopf, gestaltet sich oftmals schwierig und ist mit einem grossen Zeitaufwand verbunden, was zu relativ hohen Kosten führt und in bestimmten Gebieten, wie z.B. bei Arbeiten in Gefahrenzonen oder in der Chirurgie, zu einem erhöhten Gefahren- bzw. Komplikationspotential führt.

Gemäss dem Stand der Technik werden beschädigte Schrauben, sofern möglich, mit so genannten Schraubenausdrehern geborgen. Dies sind bohrerähnliche Werkzeuge mit einem kegelförmigen Formergewinde, dessen Einschraubrichtung der Ausdrehrichtung der jeweiligen zu bergenden Schraube entspricht. Zum Bergen der Schraube wird der Schraubenausdreher in eine in der Schraube vorhandene oder vorgängig in die Schraube eingebrachte Längsbohrung eingeschraubt, wobei sich das kegelförmige Formergewinde zunehmend tief in die Begrenzungen der Bohrung eingräbt und dadurch eine radiale Anpresskraft zwischen dem Gewinde und der Schraube erzeugt, welche im Erfolgsfall gross genug ist, um das zum Herausdrehen der Schraube erforderliche Drehmoment zu übertragen. Problematisch bei den bekannten Schraubenausdrehern ist jedoch, dass diese in einem sehr kleinen Bereich sehr grosse Spreizkräfte in die Schraube einleiten, was in vielen Fällen dazu führt, dass die Schraube in dem Bereich in welchem der Schraubenausdreher angreift, ausbricht oder ausreisst und die Bergung misslingt.

Weiter ist es aus der Chirurgie zum Bergen von beschädigten Knochenschrauben bekannt, mit einem Kronenbohrer Knochenmaterial um die zu bergende Schraube herum zu entfernen und sodann auf den so freigelegten Schraubenstumpf ein Ausdrehwerkzeug mit einem kegelförmig zulaufenden Former-Innengewinde aufzuschrauben, dessen Aufschraubrichtung der Ausdrehrichtung der jeweiligen zu bergenden Schraube entspricht. Dabei gräbt sich das kegelförmige Formergewinde zunehmend tief in die äusseren Begrenzungen des Schraubenstumpfs ein und erzeugt eine radiale Anpresskraft zwischen dem Gewinde und dem Schraubenstumpf, welche im Erfolgsfall gross genug ist, um das zum Herausdrehen der Schraube erforderliche Drehmoment zu übertragen. Hier besteht das zuvor beschriebene Problem mit den Spreizkräften nicht und es lassen sich durch den Angriff am äusseren Umfang der Schraube deutliche grössere Drehmomente übertragen. Nachteilig ist bei dieser Lösung jedoch, dass Knochenmaterial um die zu bergende Schraube herum abgetragen werden muss.

Es stellt sich deshalb die Aufgabe, technische Lösungen zur Verfügung zu stellen, welche die zuvor erwähnten Nachteile des Standes der Technik nicht aufweisen oder zumindest teilweise vermeiden.

Diese Aufgabe wird durch die Gegenstände der unabhängigen Patentansprüche gelöst.

Gemäss diesen betrifft ein erster Aspekt der Erfindung eine Schraube mit einem Schraubenschaft, dessen Aussenfläche zumindest über einen Teilbereich seiner axialen Erstreckung von einem Gewinde gebildet bzw. überzogen ist, und mit einem Schraubenkopf, welcher an einem Ende des Schraubenschafts angeordnet ist und radial über den Schraubenschaft übersteht. Die Schraube weist eine Öffnung auf, welche von der dem Schraubenschaft abgewandten Seite des Schraubenkopfes her in axialer Richtung, insbesondere zentral bezogen auf die Schraubenlängsachse, in den Schraubenkopf eindringt, den Schraubenkopf vollständig durchdringt und soweit in den Schraubenschaft eindringt, dass sie diesen zumindest teilweise axial durchsetzt. Diese Öffnung weist in dem Bereich oder in einem Teil des Bereichs, in welchem sie sich innerhalb des Schraubenschafts erstreckt, einen nicht-kreisrunden Querschnitt auf, welcher einen rotatorischen Formschluss mit einer entsprechenden Gegenkontur eines Ausdrehwerkzeugs zwecks Übertragung eines Drehmoments um die Schraubenlängsachse herum in Gewindeausdrehrichtung auf die Schraube ermöglicht, zum Ausdrehen der Schraube oder Teilen davon mit dem Ausdrehwerkzeug aus einem mit dem Schraubengewinde in Eingriff stehenden Material. Bevorzugterweise ist die erfindungsgemässe Schraube eine Knochenschraube zur Verwendung in der Chirurgie.

Durch die erfindungsgemässe Ausgestaltung der Schraube wird es möglich, auch beschädigte derartige Schrauben, z.B. mit rund gedrehtem oder abgerissenem Schraubenkopf, oder Teilstücke solcher Schrauben, d.h. Teilstücke des Schraubenschafts, in kürzester Zeit mit einem geeigneten Ausdrehwerkzeug ohne Späne und/oder Abrieb erzeugende Eingriffe aus dem mit dem Schraubengewinde in Eingriff stehenden Material herauszudrehen. Insbesondere bei Arbeiten in Gefahrenzonen oder in der Chirurgie lässt sich hierdurch das Gefahren- bzw. Komplikationspotential deutlich reduzieren.

Bevorzugterweise erstreckt sich die Öffnung innerhalb des Schraubenschafts in einem Bereich, welcher von dem Gewinde umgeben ist, und weist zumindest in diesem Bereich einen nicht-kreisrunden Querschnitt auf, zur Ermöglichung eines rotatorischen Formschlusses mit einer entsprechenden Gegenkontur eines Ausdrehwerkzeugs zwecks Übertragung eines Drehmoments um die Schraubenlängsachse herum in Gewindeausdrehrichtung auf die Schraube. Dadurch, dass die Öffnung zumindest in dem Bereich, in welchem sie vom Schraubengewinde umgeben ist, so ausgestaltet ist, dass sie einen rotatorischen Formschlusses mit einer entsprechenden Gegenkontur eines Ausdrehwerkzeugs ermöglicht, kann sichergestellt werden, dass bei einem Abreissen der Schraube eine rotatorische Krafteinleitung in das verbleibende Schraubenteilstück zumindest an dem Ort möglich ist, wo dieses Teilstück besonders fest vom umgebenden Material festgehalten wird, nämlich im Bereich des Schraubengewindes.

Mit Vorteil weist die Öffnung im gesamten Bereich, in welchem sie sich innerhalb des Schraubenschafts erstreckt, und insbesondere auch in dem Bereich, in welchem sie sich innerhalb des Schraubenkopfes erstreckt, einen nicht-kreisrunden Querschnitt auf, zur Ermöglichung eines rotatorischen Formschlusses mit einer entsprechenden Gegenkontur eines Ausdrehwerkzeugs zwecks Übertragung eines Drehmoments um die Schraubenlängsachse herum in Gewindeausdrehrichtung auf die Schraube. Hierdurch kann bei einem Abreissen der Schraube eine rotatorische Krafteinleitung in das verbleibende Schraubenteilstück an einer beliebigen axialen Position innerhalb der Öffnung erfolgen.

In einer bevorzugten Ausführungsform der Schraube weist die Öffnung in dem Bereich innerhalb des Schraubenschafts, in welchem sie den nicht-kreisrunden Querschnitt zur Ermöglichung eines rotatorischen Formschlusses mit einer entsprechenden Gegenkontur eines Ausdrehwerkzeugs aufweist, überall den gleichen Querschnitt aufweist. Die Öffnung weist also in diesem Bereich die Form eines Zylinders auf. Durch diese Ausgestaltung ergibt sich der Vorteil, dass bei einem Abreissen der Schraube im Bereich des Schraubenschafts unabhängig vom Ort des Abreissens immer der gleiche Querschnitt der Öffnung vorliegt zur Ermöglichung eines rotatorischen Formschlusses mit einer entsprechenden Gegenkontur eines Ausdrehwerkzeugs, was insbesondere für Schrauben mit zylindrischem Schaft vorteilhaft ist, weil sich so eine gleichmässige Wandstärke des Schraubenschafts realisieren und damit Schwachstellen vermeiden lassen.

Dabei haben in einer ersten bevorzugten Variante die Querschnitte der Öffnung in diesem Bereich überall die gleiche rotatorische Ausrichtung bezogen auf die Schraubenlängsachse, so dass die Öffnung in diesem Bereich die Form eines geraden Zylinders aufweist. Derartige Öffnungskonturen lassen sich einfach und kostengünstig herstellen, insbesondere dann, wenn sich die Öffnung als Durchgangsöffnung vollständig durch die Schraube hindurch erstreckt.

In einer zweiten bevorzugten Variante sind die Querschnitte der Öffnung in diesem Bereich in Schraubenlängsrichtung bevorzugterweise gleichmässig fortschreitend um die Schraubenlängsachse herum gegeneinander verdreht, so dass die Öffnung in diesem Bereich die Form eines bevorzugterweise gleichmässig in Schraubenausdrehrichtung tordierten Zylinders aufweist. Diese Variante ergibt insbesondere bei kleinen Öffnungsquerschnitten den Vorteil, dass eine grössere axiale Erstreckung der Öffnung der Übertragung eines Ausdrehmoments in die Schraube dienen kann, weil durch die derart tordierte Kontur der Öffnung der Torsionsneigung der entsprechenden Gegenkontur des Ausdrehwerkzeugs unter dem Ausdrehmoment Rechnung getragen werden kann.

In einer anderen bevorzugten Ausführungsform der Schraube weist die Öffnung in dem Bereich innerhalb des Schraubenschafts, in welchem sie den nicht-kreisrunden Querschnitt zur Ermöglichung eines rotatorischen Formschlusses mit einer entsprechenden Gegenkontur eines Ausdrehwerkzeugs aufweist, überall die gleiche Querschnittsform auf, jedoch mit sich in Schraubenlängsrichtung zum Schraubenkopf hin bevorzugterweise gleichmässig vergrössernden Querschnittsflächen. Hierdurch ergibt sich in diesem Bereich eine kegel- oder pyramidenähnliche Form der Öffnung. Durch diese Ausgestaltung ergibt sich zumindest bei Schrauben mit konischem Schaft der Vorteil, dass die Schraube derartig ausgebildet werden kann, dass sich der Querschnitt der Öffnung mit zunehmendem Schaftdurchmesser vergrössert, so dass bei einem Abreissen der Schraube im Bereich des Schraubenschafts unabhängig vom Ort des Abreissens immer ein maximaler Querschnitt der Öffnung bezogen auf den Schraubendurchmesser bereitgestellt werden kann zur Ermöglichung eines rotatorischen Formschlusses mit einer entsprechenden Gegenkontur eines Ausdrehwerkzeugs, wodurch jeweils eine optimale Drehmomenteinleitung in den Schraubenschaft ermöglicht wird.

In einer weiteren bevorzugten Ausführungsform der erfindungsgemässen Schraube durchsetzt die Öffnung den Schraubenschaft vollständig, d.h. sie erstreckt sich als Durchgangsöffnung vollständig durch die Schraube hindurch, so dass sie an dem Ende des Schraubenschafts, welches dem Schraubenkopf abgewandt ist, aus dem Schraubenschaft austritt. Derartige Schrauben lassen sich besonders einfach und kostengünstig fertigen und können derartig ausgebildet werden, dass bei einem Abreissen der Schraube im Bereich des Schraubenschafts unabhängig vom Ort des Abreissens ein nicht-kreisrunder Querschnitt der Öffnung vorliegt, in den eine entsprechende Gegenkontur eines Ausdrehwerkzeugs eingesetzt werden kann zur Ermöglichung eines rotatorischen Formschlusses mit dem verbliebenen Schraubenschaftteil.

Mit Vorteil weist die Öffnung der erfindungsgemässen Schraube in dem Bereich innerhalb des Schraubenschafts, in welchem sie den nicht-kreisrunden Querschnitt zur Ermöglichung eines rotatorischen Formschlusses mit einer entsprechenden Gegenkontur eines Ausdrehwerkzeugs aufweist, einen vieleckigen Querschnitt auf, und zwar bevorzugterweise den Querschnitt eines gleichmässigen Quadrats oder Sechsecks (z.B. Inbus), oder den Querschnitt eines Aussen-Vielrunds, bevorzugterweise eines gleichmässigen Aussen-Sechrunds (z.B. Torx). Mit anderen Worten ausgedrückt bilden also die diese Öffnung umgebenden Begrenzungwandungen der Schraube ein Innenvieleck, und zwar bevorzugterweise ein gleichmässiges Innen-Viereck oder Innen-Sechseck (Inbus), oder einen Innen-Vielrund, bevorzugterweise einen gleichmässigen Innen-Sechrund (Torx). Profile mit derartigen Querschnittsformen sind zur Übertragung von Ein- bzw. Ausdrehmomenten auf Schrauben besonders gut geeignet und heute in diesem Fachgebiet allgemein etabliert.

In noch einer weiteren bevorzugten Ausführungsform der Schraube ist die Öffnung der Schraube im Bereich des Schraubenkopfes gegenüber dem Bereich der Öffnung, welcher sich innerhalb des Schraubenschafts erstreckt, erweitert und bildet in diesem erweiterten Bereich eine Mitnehmerkontur zum Eingreifen eines Eindreh- bzw. Ausdrehwerkzeugs, bevorzugterweise eine Kreuzschlitz-, Innen-Sechskant- oder Innen-Sechsrund-Kontur, zur Übertragung eines Drehmoments auf den Kopf der Schraube zum Ein- bzw. Ausdrehen derselben. Auf diese Weise kann der gegenüber dem Schaft erweiterte Durchmesser des Schraubenkopfes genutzt werden, eine möglichst optimale Mitnehmerkontur zum Ein- bzw. Ausdrehen der Schraube bereitzustellen, so dass lediglich für den Fall eines Versagens dieser Mitnehmerkontur oder eines Abreissens der Schraube mit einem geeigneten Ausdrehwerkzeug in die im Querschnitt nicht-kreisrunde Kontur der Öffnung im Bereich des Schraubenschafts eingegriffen werden muss, zum Ausdrehen der beschädigten Schraube.

Bevorzugterweise ist die erfindungsgemässe Schraube aus rostfreiem Stahl, Titan, einer Titanlegierung oder aus einem Sinterwerkstoff gebildet, was insbesondere dann von Vorteil ist, wenn sie in der Chirurgie zum Einsatz kommen soll bzw. spezielle Festigkeiten und/- oder Konturen aufweisen soll.

Wird die Schraube durch Selektives Laserschmelzen, auch bekannt als "Lasercusing", "Direct Metal Laser Sintering" oder "Selective Laser Melting", hergestellt, was für bestimmte Ausführungsformen bevorzugt ist, so lassen sich auch bezüglich der Öffnungsgeometrie sehr komplizierte Schraubenkonturen auf wirtschaftliche Weise realsieren.

Ein zweiter Aspekt der Erfindung betrifft ein Werkzeug zum Ein- bzw. Ausdrehen einer Schraube gemäss dem ersten Aspekt der Erfindung. Das Werkzeug umfasst einen Mitnehmerdorn mit einem nicht-kreisrunden Querschnitt zum rotatorisch formschlüssigen Eingreifen in den nicht-kreisrunden Querschnitt der Öffnung der Schraube innerhalb des Schraubenschafts zwecks Übertragung eines Drehmoments um die Schraubenlängsachse herum auf die Schraube. Der Mitnehmerdorn wird von einem Führungsschaft zum Führen des Werkzeugs mit einer Hand des Bedieners getragen, und steht dabei bevorzugterweise zentral von einem Ende desselben ab. Am anderen Ende des Führungsschaft ist ein Griffteil angeordnet, zum Ergreifen und Drehen des Werkzeugs mit der anderen Hand des Bedieners zwecks Ein- bzw. Ausschrauben der Schraube mit dem Werkzeug. An dem Endes des Führungsschafts, von welchem der Mitnehmerdorn absteht, bildet dessen Stirnseite eine Anschlagschulter zum Anschlagen des Schraubenkopfes bei bestimmungsgemäss in die Öffnung der Schraube eingeführtem Mitnehmerdorn. Mit einem derartigen Werkzeug lassen sich beschädigte Schrauben gemäss dem ersten Aspekt der Erfindung, z.B. mit beschädigtem oder abgerissenen Kopf, bergen.

In einer bevorzugten Ausführungsform des Werkzeugs ist der Übergang vom Mitnehmerdorn auf den Führungsschaft gegenüber dem übrigen Mitnehmerdorn erweitert und bildet eine Kontur zum Eingreifen in eine entsprechende Mitnehmerkontur im Schraubenkopf, z.B. eine Kontur zum Eingreifen in eine als Kreuzschlitz, Innen-Sechskant oder Innen-Sechsrund ausgebildete Mitnehmerkontur im Schraubenkopf. Derartige Werkzeuge eignen sich als Ein-und Ausdrehwerkzeug für entsprechende erfindungsgemässe Schrauben und weisen gegenüber herkömmlichen Ein- bzw. Ausdrehwerkzeugen, die lediglich eine Kontur zum Eingreifen in eine entsprechende Mitnehmerkontur im Schraubenkopf aufweisen, wie z.B. Kreuzschlitz-Schraubendreher oder Torx- bzw. Inbusschlüssel, den Vorteil auf, dass die Schraube auf dem Mitnehmerdorn geführt ist, was zumindest das Ausrichten und Einsetzen der Schraube in ein Einschraubloch an schwer zugänglichen Stellen deutlich erleichtert.

In einer weiteren bevorzugten Ausführungsform des Werkzeugs ist der Mitnehmerdorn als Profilstab mit einem nicht-kreisrunden Querschnitt ausgebildet, und zwar bevorzugterweise als Aussen-Sechsrund-Profilstab, z.B. als Torx-Profilstab.

In einer anderen bevorzugten Ausführungsform des Werkzeugs ist der Mitnehmerdorn mit einer kegel- oder pyramidenähnlichen Form mit nicht-kreisrundem Querschnitt, deren Querschnittsfläche zum Führungsschaft hin gesehen zunimmt, ausgebildet, und zwar bevorzugterweise als pyramidenförmig verlaufender Vierkant.

Je nachdem, für welche Schraubenvariante das Werkzeug zur Anwendung kommt, ist die eine oder die andere Ausführungsform vorgesehen.

Ein dritter Aspekt der Erfindung betrifft ein Set umfassend eine Schraube gemäss dem ersten Aspekt der Erfindung und ein Werkzeug gemäss dem zweiten Aspekt der Erfindung, wobei der Mitnehmerdorn des Werkzeugs derartig ausgebildet ist, dass er im bestimmungsgemäss in die Öffnung der Schraube eingeführten Zustand bei an der Anschlagschulter des Werkzeugs anstehendem Schraubenkopf mit sämtlichen innerhalb des Schraubenschafts gebildeten Bereichen der Öffnung mit nicht-kreisrundem Querschnitt einen rotatorischen Formschluss bildet. Ein solches Set bieten den Vorteil, dass bei einem Abreissen der Schraube auch tief im Einschraubloch zurückgebliebene Teile des Schraubenschafts mit dem Werkzeug geborgen werden können, da dieses über einen in jedem Fall ausreichend langen Mitnehmerdorn verfügt.

In einer bevorzugten Ausführungsform des Sets ist die Öffnung der Schraube im Bereich des Schraubenkopfes erweitert gegenüber den Bereich der Öffnung, welcher sich innerhalb des Schraubenschafts erstreckt, und bildet in diesem erweiterten Bereich eine Mitnehmerkontur, in welche eine entsprechende, am Werkzeug des Sets im Bereich des Übergangs vom Mitnehmerdorn auf den Führungsschaft gebildete Kontur eingreifen kann, zur Übertragung eines Drehmoments vom Werkzeug auf die Schraube zum Ein- bzw. Ausdrehen derselben. Die Mitnehmerkontur ist bevorzugterweise als Kreuzschlitz, Innen-Sechskant oder Innen-Sechsrund ausgebildet.

Ein vierter Aspekt der Erfindung betrifft ein Verfahren zur spanlosen Entfernung einer insbesondere beschädigten Schraube gemäss dem ersten Aspekt der erfindung aus einem zumindest mit einem Teil des Schraubengewindes in Eingriff stehenden Material. Das Verfahren umfasst die Schritte:
a) Bereitstellen eines Ausdrehwerkzeugs, bevorzugterweise gemäss dem zweiten Aspekt der Erfindung, mit einer Werkzeugkontur, welche einen rotatorischen Formschluss in Gewindeausdrehrichtung der Schraube mit den innerhalb des Schraubenschafts gebildeten Bereichen der Öffnung mit nicht-kreisrundem Querschnitt ermöglicht;
b) Einführen des Ausdrehwerkzeugs in die Öffnung derartig, dass mit den innerhalb des Schraubenschafts gebildeten Bereichen der Öffnung mit nicht-kreisrundem Querschnitt ein rotatorischer Formschluss in Gewindeausdrehrichtung der Schraube erzeugt wird; und
c) Ausdrehen der Schraube aus dem Material durch Drehen des Ausdrehwerkzeugs um die Längsachse herum in Ausdrehrichtung der Schraube.

Unter einer "spanlosen" Entfernung der Schraube bzw. eines Teils davon aus einem zumindest mit einem Teil des Schraubengewindes in Eingriff stehenden Material wird hier ein Entfernen verstanden, bei welchem keine zerspanenden Bearbeitungsschritte erforderlich sind, weder an der Schraube selbst noch an dem mit der Schraube in Kontakt stehenden Material. Ein etwaiges Abplatzen von Material beim Herausdrehen der Schraube oder eines Teils davon aus dem Material wird hier nicht als zerspanender Bearbeitungsschritt verstanden.

Bei der Ausführung des erfindungsgemässen Verfahrens, welches eine zerspanungs- bzw. abrieblose Bergung auch beschädigter und insbesondere abgerissener Schrauben ermöglicht, treten die Vorteile der Erfindung besonder deutlich zu Tage.

In einer bevorzugten Ausführungsform des Verfahrens wird die Schraube aus einem Knochen herausgedreht, insbesondere aus einem Knochen eines menschlichen oder tierischen Patienten. Insbesondere bei letztgenannten Verfahrensvarianten ergeben sich grosse Vorteile, da sich mit diesen das Gefahren- bzw. Komplikationspotential bei einem derartigen chirurgischen Eingriff deutlich reduzieren lässt und damit auch die Eingriffszeit und Eingriffskosten.

Weitere bevorzugte Ausführungen der Erfindung ergeben sich aus den abhängigen Ansprüchen sowie aus der nun folgenden Beschreibung anhand der Figuren. Dabei zeigen:
die Figuren 1a bis 1d verschiedene Ansichten und Schnitte einer ersten erfindungsgemässen Schraube;
die Figuren 2a bis 2d verschiedene Ansichten und Schnitte eines ersten erfindungsgemässen Werkzeugs zum Ein- und Ausdrehen der Schraube aus den Figuren 1a bis 1d;
die Figuren 3a bis 3d verschiedene Ansichten und Schnitte eines ersten erfindungsgemässen Sets gebildet aus einer Schraube gemäss den Figuren 1a bis 1d und einem Werkzeug gemäss den Figuren 2a bis 2d;
die Figuren 4a bis 4d verschiedene Ansichten und Schnitte einer zweiten erfindungsgemässen Schraube;
die Figuren 5a und 5b verschiedene Ansichten eines zweiten erfindungsgemässen Werkzeugs zum Ein- und Ausdrehen der Schraube aus den Figuren 4a bis 4d; und
die Figuren 6a bis 6c verschiedene Ansichten und einen Längsschnitt eines zweiten erfindungsgemässen Sets gebildet aus einer Schraube gemäss den Figuren 4a bis 4d und einem Werkzeug gemäss den Figuren 5a und 5b.

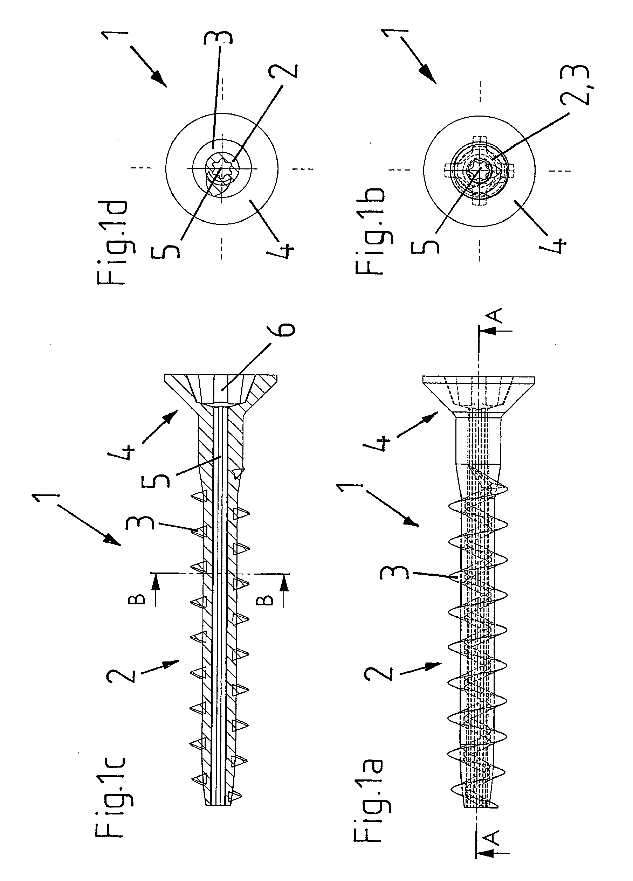

Die Figuren 1a bis 1d zeigen eine Seitenansicht (Fig. 1a) und eine Draufsicht auf die Spitze (Fig. 1b) einer ersten erfindungsgemässen Knochenschraube 1 sowie einen Längsschnitt durch die Schraube 1 entlang der Linie A-A in Fig. 1a (Fig. 1c) und einen Querschnitt durch die Schraube 1 entlang der Linie B-B in Fig. 1c (Fig. 1d).

Wie zu erkennen ist, weist die Schraube 1 einen über einen Grossteil seiner Erstreckung im Wesentlichen zylindrisch ausgebildeten Schraubenschaft 2 auf, dessen Aussenfläche zumindest über den gesamten zylindrischen Bereich von einem Gewinde 3 gebildet bzw. überzogen ist. Weiter weist die Schraube 1 einen kegelstumpfförmigen Schraubenkopf 4 (Senkkopfschraube) auf, welcher an einem Ende des Schraubenschafts 2 angeordnet ist und radial über den Schraubenschaft 2 übersteht.

Die Schraube 1 ist über ihre gesamte axiale Erstreckung von einer zentralen Öffnung 5 durchsetzt, welche innerhalb des Schraubenschafts 2 einen gleichmässigen Querschnitt in Form eines gleichmässigen Aussen-Sechsrunds aufweist und sich im Bereich des Schraubenkopfes 4 zu einer Aufnahmeöffnung 6 (anspruchsgemässe Mitnehmerkontur) für eine Kreuzschlitzspitze eines Ein- bzw. Ausschraubwerkzeugs erweitert.

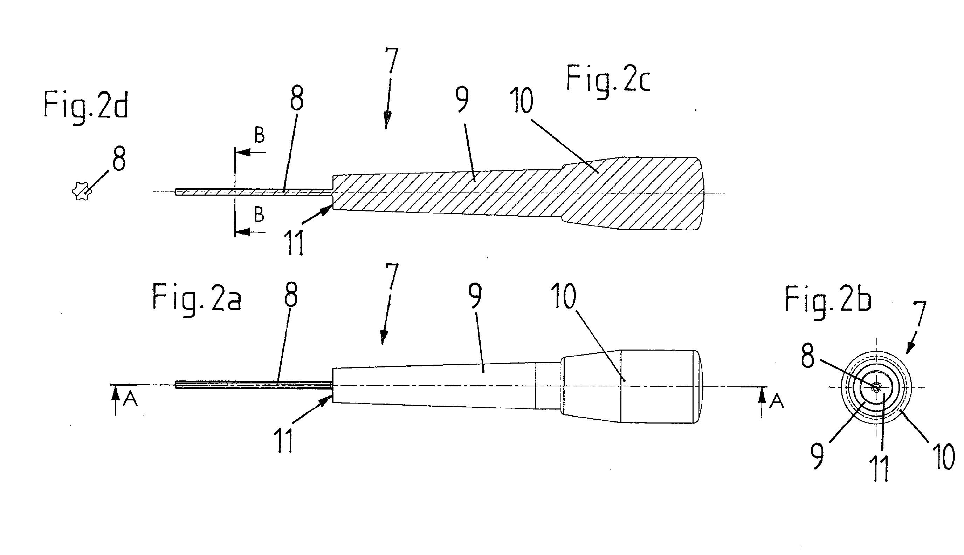

Die Figuren 2a bis 2d zeigen eine Seitenansicht (Fig. 2a) und eine Draufsicht auf die Spitze (Fig. 2b) eines ersten erfindungsgemässen Werkzeugs 7 zum Ein-und Ausdrehen der Schraube 1 aus den Figuren 1a bis 1d sowie einen Längsschnitt durch das Werkzeug 7 entlang der Linie A-A in Fig. 2a (Fig. 2c) und einen Querschnitt durch das Werkzeug 7 entlang der Linie B-B in Fig. 2c (Fig. 2d).

Wie zu erkennen ist, umfasst das Werkzeug 7 einen Mitnehmerdorn 8, der als Profilstab mit dem Aussen-Sechsrund-Profil der Öffnung 5 der Schraube 1 ausgebildet ist.

Weiter umfasst das Werkzeug 7 einen Führungsschaft 9 zum Führen des Werkzeugs 7 mit einer Hand des Bedieners. Der Mitnehmerdorn 8 steht zentral von einem Ende des Führungsschafts 9 ab. An dem anderen Ende des Führungsschafts 9 ist ein Griffteil 10 angeordnet, zum Ergreifen und Drehen des Werkzeugs 7 mit der anderen Hand des Bedieners zwecks Ein- bzw. Ausschrauben der Schraube 1 mit dem Werkzeug 7.

An demjenigen Ende des Führungsschafts 9, an welchem der Mitnehmerdorn 8 absteht, bildet die Stirnfläche desselben eine Anschlagschulter 11 zum Anschlagen des Schraubenkopfes 4 bei bestimmungsgemäss in die Öffnung 5 der Schraube 1 eingeführtem Mitnehmerdorn 8.

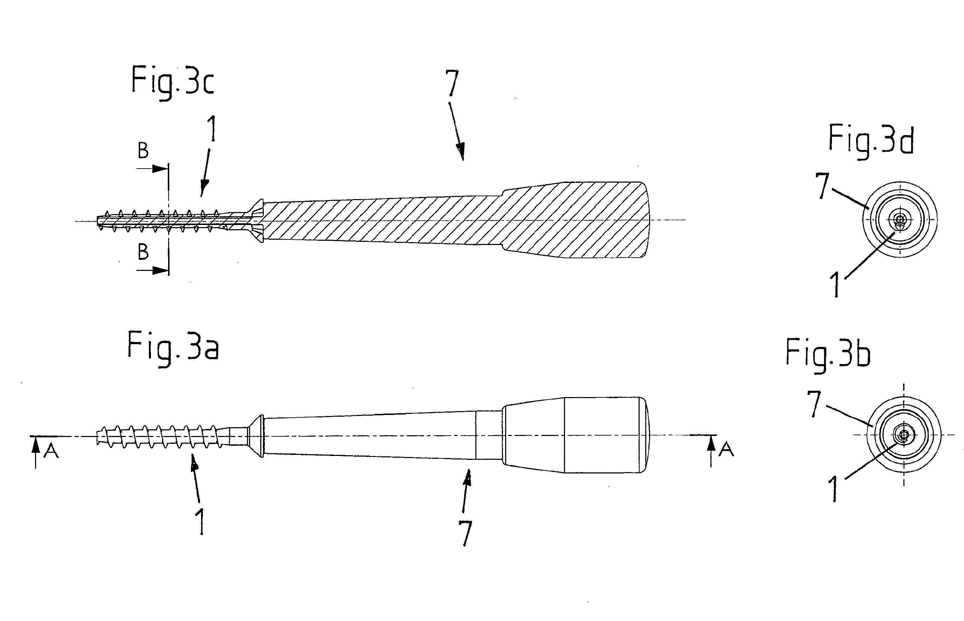

Diese Situation ist aus den Figuren 3a bis 3d ersichtlich, welche eine Seitenansicht (Fig. 3a) und eine Draufsicht auf die Spitze der Schraube (Fig. 3b) eines entsprechend zusammengefügten ersten erfindungsgemässen Sets gebildet aus einer Schraube 1 gemäss den Figuren 1a bis 1d und einem Werkzeug 7 gemäss den Figuren 2a bis 2d zeigen sowie einen Längsschnitt durch das Set entlang der Linie A-A in Fig. 3a (Fig. 3c) und einen Querschnitt durch das Set entlang der Linie B-B in Fig. 3c (Fig. 3d).

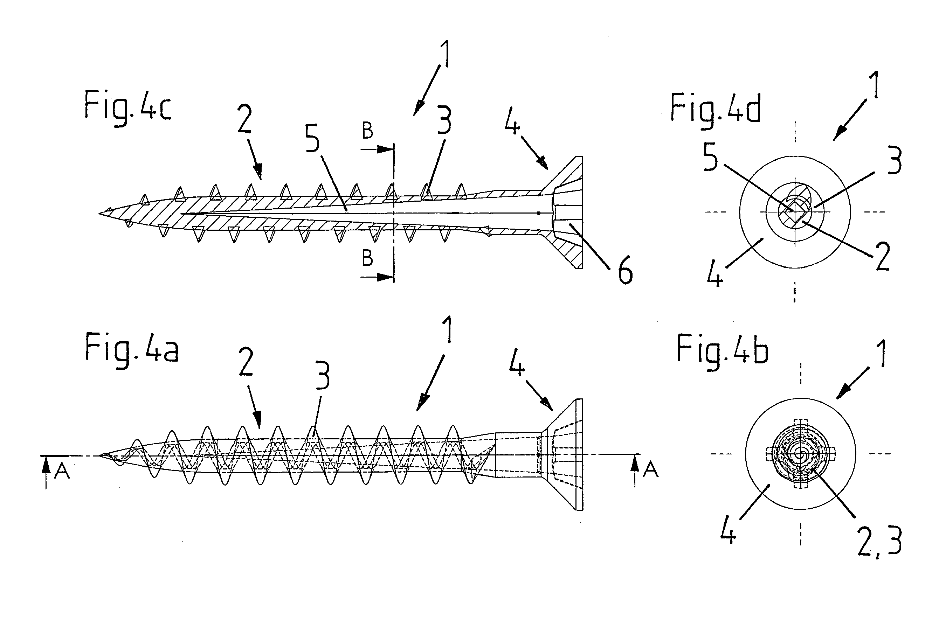

Die Figuren 4a bis 4d zeigen eine Seitenansicht (Fig. 4a) und eine Draufsicht auf die Spitze (Fig. 4b) einer zweiten erfindungsgemässen Knochenschraube 1 sowie einen Längsschnitt durch die Schraube 1 entlang der Linie A-A in Fig. 1a (Fig. 4c) und einen Querschnitt durch die Schraube 1 entlang der Linie B-B in Fig. 4c (Fig. 4d).

Diese zweite erfindungsgemässe Schraube 1 unterscheidet sich äusserlich von der in den Figuren 1a bis 1d dargestellten ersten erfindungsgemässen Schraube im Wesentlichen dadurch, dass ihr Schraubenschaft 2 an seinem dem Schraubenkopf 4 abgewandten Ende spitz ausläuft.

Wie anhand der Schnittdarstellungen zu erkennen ist, besteht ein weiterer wesentlicher Unterschied zur ersten erfindungsgemässen Schraube zudem darin, dass diese zweite erfindungsgemässe Schraube 1 nicht über ihre gesamte axiale Erstreckung von einer zentralen Öffnung durchsetzt ist, sondern eine zentrale Öffnung 5 aufweist, die vom Schraubenkopf 4 her axial in die Schraube 1 eindringt und diese bis kurz vor der Schraubenspitze durchsetzt. Auch weist diese Öffnung 5 nicht einen über ihre Erstreckung innerhalb des Schraubenschafts 2 identischen Querschnitt in Form eines gleichmässigen Aussen-Sechsrunds auf, sondern weist in diesem Bereich überall eine quadratische Querschnittsform mit in Schraubenlängsrichtung zum Schraubenkopf 4 hin gleichmässig sich vergrössernden Querschnittsflächen auf, derart, dass die Öffnung in diesem Bereich eine pyramidenähnliche Form aufweist.

Alle übrigen Ausgestaltungen der Schraube sind identisch zu denjenigen der in den Figuren 1a bis 1d gezeigten Schraube.

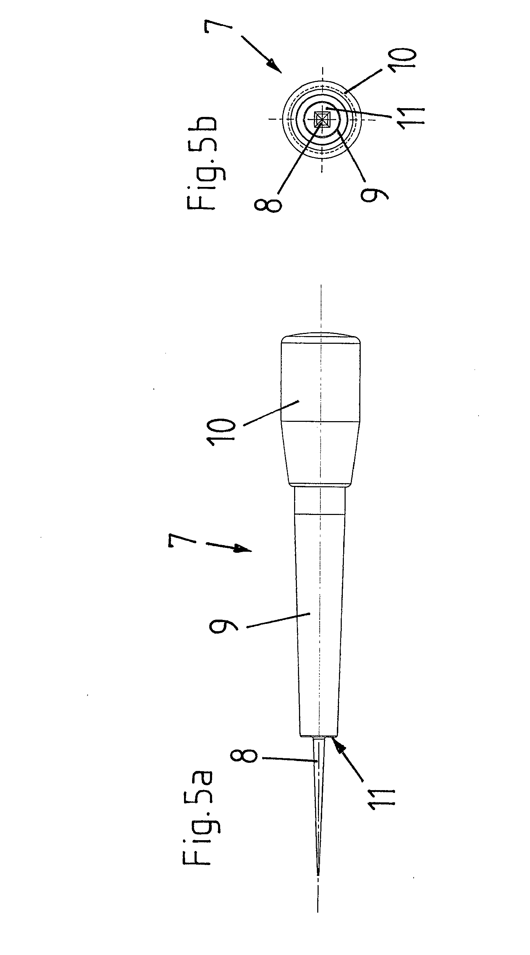

Die Figuren 5a und 5b zeigen eine Seitenansicht (Fig. 5a) und eine Draufsicht auf die Spitze (Fig. 5b) eines zweiten erfindungsgemässen Werkzeugs 7.

Dieses Werkzeug ist ausgestaltet zum Ein- und Ausdrehen der Schraube 1 aus den Figuren 4a bis 4d und unterscheidet sich entsprechend von dem in den Figuren 2a bis 2d gezeigten Werkzeug in der Ausgestaltung des Mitnehmerdorns 8. Wie zu erkennen ist, ist dieser hier nicht als Profilstab mit einem Aussen-Sechsrund-Profil ausgebildet, sondern als Mitnehmerdorn 8 mit der pyramidenähnlichen Vierkant-Form der Öffnung 5 der Schraube 1.

Alle übrigen Ausgestaltungen des Werkzeugs sind identisch zu denjenigen des in den Figuren 2a bis 2d gezeigten Werkzeugs.

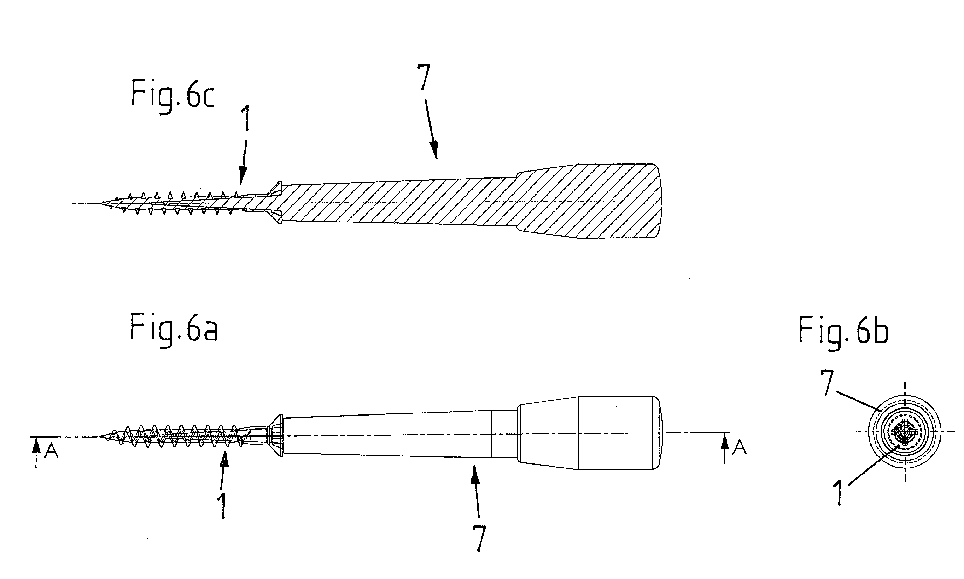

Die Figuren 6a bis 6c zeigen eine Seitenansicht (Fig. 6a) und eine Draufsicht auf die Spitze der Schraube (Fig. 6b) eines bestimmungsgemäss zum Ein- bzw. Ausdrehen der Schraube 1 zusammengefügten zweiten erfindungsgemässen Sets gebildet aus einer Schraube 1 gemäss den Figuren 4a bis 4d und einem Werkzeug 7 gemäss den Figuren 5a und 5b, sowie einen Längsschnitt durch das Set entlang der Linie A-A in Fig. 6a (Fig. 6c).

Während in der vorliegenden Anmeldung bevorzugte Ausführungen der Erfindung beschrieben sind, ist klar darauf hinzuweisen, dass die Erfindung nicht auf diese beschränkt ist und auch in anderer Weise innerhalb des Umfangs der folgenden Patentansprüche ausgeführt werden kann.

## Patentansprüche

1. Schraube (1), insbesondere Knochenschraube zur Verwendung in der Chirurgie, umfassend einen Schraubenschaft (2), dessen Aussenfläche zumindest über einen Teilbereich seiner axialen Erstreckung von einem Gewinde (3) gebildet bzw. überzogen ist, und einen Schraubenkopf (4), welcher an einem Ende des Schraubenschafts (2) angeordnet ist und radial über den Schraubenschaft (2) übersteht, wobei die Schraube (1) eine Öffnung (5) aufweist, welche von der dem Schraubenschaft (2) abgewandten Seite des Schraubenkopfes (4) her in axialer Richtung, insbesondere zentral bezogen auf die Schraubenlängsachse, in den Schraubenkopf (4) eindringt, diesen vollständig durchsetzt und in den Schraubenschaft (2) eindringt, welchen sie zumindest teilweise durchsetzt, und wobei die Öffnung (5) in einem Bereich, welcher sich innerhalb des Schraubenschafts (2) erstreckt, einen nicht-kreisrunden Querschnitt aufweist, zur Ermöglichung eines rotatorischen Formschlusses mit einer entsprechenden Gegenkontur (8) eines Ausdrehwerkzeugs (7) zwecks Übertragung eines Drehmoments um die Schraubenlängsachse herum in Gewindeausdrehrichtung auf die Schraube (1).

2. Schraube (1) nach Anspruch 1, wobei sich die Öffnung (5) innerhalb des Schraubenschafts (2)in einem Bereich erstreckt, welcher von dem Gewinde (3) umgeben ist, und zumindest in diesem Bereich einen nicht-kreisrunden Querschnitt aufweist, zur Ermöglichung eines rotatorischen Formschlusses mit einer entsprechenden Gegenkontur (8) eines Ausdrehwerkzeugs (7) zwecks Übertragung eines Drehmoments um die Schraubenlängsachse herum in Gewindeausdrehrichtung auf die Schraube (1).

3. Schraube (1) nach einem der vorangehenden Ansprüche, wobei die Öffnung (5) im gesamten Bereich, in welchem sie sich innerhalb des Schraubenschafts (2) erstreckt, und insbesondere auch in dem Bereich, in welchem sie sich innerhalb des Schraubenkopfes (4) erstreckt, einen nicht-kreisrunden Querschnitt aufweist, zur Ermöglichung eines rotatorischen Formschlusses mit einer entsprechenden Gegenkontur (8) eines Ausdrehwerkzeugs (7) zwecks Übertragung eines Drehmoments um die Schraubenlängsachse herum in Gewindeausdrehrichtung auf die Schraube (1).

4. Schraube (1) nach einem der vorangehenden Ansprüche, wobei die Öffnung (5) in dem Bereich innerhalb des Schraubenschafts (2), in welchem sie den nicht-kreisrunden Querschnitt aufweist, überall den gleichen Querschnitt aufweist, derart, dass die Öffnung (5) in diesem Bereich die Form eines Zylinders aufweist.

5. Schraube (1) nach Anspruch 4, wobei die Querschnitte der Öffnung (5) in dem Bereich innerhalb des Schraubenschafts (2), in welchem sie den nicht-kreisrunden Querschnitt aufweist, überall die gleiche rotatorische Ausrichtung bezogen auf die Schraubenlängsachse aufweisen, derart, dass die Öffnung (5) in diesem Bereich die Form eines geraden Zylinders aufweist.

6. Schraube (1) nach Anspruch 4, wobei die Querschnitte der Öffnung (5) in dem Bereich innerhalb des Schraubenschafts (2), in welchem sie den nicht-kreisrunden Querschnitt aufweist, in Schraubenlängsrichtung insbesondere gleichmässig fortschreitend um die Schraubenlängsachse herum gegeneinander verdreht sind, derart, dass die Öffnung (5) in diesem Bereich die Form eines insbesondere gleichmässig in Schraubenausdrehrichtung tordierten Zylinders aufweist.

7. Schraube (1) nach einem der Ansprüche 1 bis 3, wobei die Öffnung (5) in dem Bereich innerhalb des Schraubenschafts (2), in welchem sie den nicht-kreisrunden Querschnitt aufweist, überall die gleiche Querschnittsform aufweist, mit in Schraubenlängsrichtung zum Schraubenkopf (4) hin insbesondere gleichmässig sich vergrössernden Querschnittsflächen, derart, dass die Öffnung (5) in diesem Bereich eine kegel- oder pyramidenähnliche Form aufweist.

8. Schraube (1) nach einem der vorangehenden Ansprüche, wobei die Öffnung (5) den Schraubenschaft (2) vollständig durchsetzt, so dass sie an dem Ende des Schraubenschafts (2), welches dem Schraubenkopf (4) abgewandt ist, aus dem Schraubenschaft (2) austritt.

9. Schraube (1) nach einem der vorangehenden Ansprüche, wobei die Öffnung (5) in dem Bereich innerhalb des Schraubenschafts (2), in welchem sie den nicht-kreisrunden Querschnitt aufweist, einen vieleckigen Querschnitt aufweist, insbesondere den Querschnitt eines gleichmässigen Quadrats oder Sechsecks aufweist, oder den Querschnitt eines Aussen-Vielrunds, insbesondere eines gleichmässigen Aussen-Sechrunds.

10. Schraube (1) nach einem der vorangehenden Ansprüche, wobei die Öffnung (5) im Bereich des Schraubenkopfes (4) erweitert ist gegenüber dem Bereich, welcher sich innerhalb des Schraubenschafts (2) erstreckt, und in diesem erweiterten Bereich eine Mitnehmerkontur (6) zum Eingreifen eines Eindreh- bzw. Ausdrehwerkzeugs bildet, insbesondere eine Kreuzschlitz-, Innen-Sechskant-oder Innen-Sechsrund-Kontur, zur Übertragung eines Drehmoments auf die Schraube (1) zum Ein- bzw. Ausdrehen derselben.

11. Schraube (1) nach einem der vorangehenden Ansprüche, wobei die Schraube (1) aus rostfreiem Stahl, Titan, einer Titanlegierung oder aus einem Sinterwerkstoff besteht.

12. Schraube (1) nach einem der vorangehenden Ansprüche, wobei die Schraube (1) durch Selektives Laserschmelzen (Lasercusing) hergestellt ist.

13. Werkzeug (7) zum Ein- bzw. Ausdrehen einer Schraube (1) nach einem der vorangehenden Ansprüche, umfassend
a) einen Mitnehmerdorn (8) mit einem nicht-kreisrunden Querschnitt zum rotatorisch formschlüssigen Eingreifen in den nicht-kreisrunden Querschnitt der Öffnung (5) der Schraube (5) innerhalb des Schraubenschafts (2) zwecks Übertragung eines Drehmoments um die Schraubenlängsachse herum auf die Schraube (1);
b) einen Führungsschaft (9) zum Führen des Werkzeugs (7) mit einer ersten Hand des Bedieners, von dessen erstem Ende der Mitnehmerdorn (8) insbesondere zentral absteht; und
c) einen Griffteil (10) angeordnet an dem zweiten Ende des Führungsschafts (9), zum Ergreifen und Drehen des Werkzeugs (7) mit der zweiten Hand des Bedieners zwecks Ein- bzw. Ausschrauben der Schraube (1) mit dem Werkzeug (7),
wobei die Stirnseite des ersten Endes des Führungsschafts (9) eine Anschlagschulter (11) bildet, zum Anschlagen des Schraubenkopfes (4) bei bestimmungsgemäss in die Öffnung (5) der Schraube (1) eingeführtem Mitnehmerdorn (8).

14. Werkzeug nach Anspruch 13, wobei der Übergang vom Mitnehmerdorn auf den Führungsschaft gegenüber dem übrigen Mitnehmerdorn erweitert ist und eine Kontur zum Eingreifen in eine entsprechende Mitnehmerkontur im Schraubenkopf bildet, insbesondere eine Kontur zum Eingreifen in eine als Kreuzschlitz, Innen-Sechskant oder Innen-Sechsrund ausgebildete Mitnehmerkontur im Schraubenkopf.

15. Werkzeug (7) nach einem der Ansprüche 13 bis 14, wobei der Mitnehmerdorn (8) als Profilstab mit einem nicht-kreisrunden Querschnitt ausgebildet ist, insbesondere als Aussen-Sechsrund-Profilstab.

16. Werkzeug (7) nach einem der Ansprüche 13 bis 14, wobei der Mitnehmerdorn (8) mit einer kegel- oder pyramidenähnlichen Form mit nicht-kreisrundem Querschnitt, deren Querschnittsfläche zum Führungsschaft (9) hin gesehen zunimmt, ausgebildet ist, insbesondere als pyramidenförmig verlaufender Vierkant.

17. Set umfassend eine Schraube (1) nach einem der Ansprüche 1 bis 12 und ein Werkzeug (7) nach einem der Ansprüche 13 bis 16, wobei der Mitnehmerdorn (8) des Werkzeugs (7) derartig ausgebildet ist, dass er im bestimmungsgemäss in die Öffnung (5) der Schraube (1) eingeführten Zustand bei an der Anschlagschulter (11) des Werkzeugs (7) anstehendem Schraubenkopf (4) mit sämtlichen innerhalb des Schraubenschafts (2) gebildeten Bereichen der Öffnung (5) mit nicht-kreisrundem Querschnitt einen rotatorischen Formschluss bildet.

18. Set nach Anspruch 17 mit einer Schraube nach Anspruch 10 und einem Werkzeug nach Anspruch 14, wobei der Übergang vom Mitnehmerdorn auf den Führungsschaft eine Kontur zum Eingreifen in eine entsprechende Mitnehmerkontur im Kopf der Schraube bildet, insbesondere eine Kontur zum Eingreifen in eine als Kreuzschlitz, Innen-Sechskant oder Innen-Sechsrund ausgebildete Mitnehmerkontur im Schraubenkopf.

19. Verfahren zur spanlosen Entfernung einer insbesondere beschädigten Schraube (1) nach einem der Ansprüche 1 bis 12 aus einem zumindest mit einem Teil des Schraubengewindes (3) in Eingriff stehenden Material, umfassend die Schritte:
a) Bereitstellen eines Ausdrehwerkzeugs (7), insbesondere nach einem der Ansprüche 13 bis 18, mit einer Werkzeugkontur (8), welche einen rotatorischen Formschluss in Gewindeausdrehrichtung der Schraube (1) mit den innerhalb des Schraubenschafts (2) gebildeten Bereichen der Öffnung (5) mit nicht-kreisrundem Querschnitt ermöglicht;
b) Einführen des Ausdrehwerkzeugs (7) in die Öffnung (5) derartig, dass mit den innerhalb des Schraubenschafts (2) gebildeten Bereichen der Öffnung (5) mit nicht-kreisrundem Querschnitt ein rotatorischer Formschluss in Gewindeausdrehrichtung der Schraube (1) erzeugt wird; und
c) Ausdrehen der Schraube (1) aus dem Material durch Drehen des Ausdrehwerkzeugs (7) um die Längsachse herum in Ausdrehrichtung der Schraube (1).

20. Verfahren nach Anspruch 19, wobei die Schraube (1) aus einem Knochen herausgedreht wird, insbesondere aus einem Knochen eines menschlichen oder tierischen Patienten.
